# EUROPEAN PATENT SPECIFICATION

(11) **EP 1 804 909 B1**
(45) Date of publication and mention of the grant of the patent: **12.08.2009**
(21) Application number: 05785113.1
(22) Date of filing: 09.09.2005
(51) Int. Cl.: A61N 1/32

(54) **APPARATUS FOR TREATMENT OF DIABETES IN A MAMMAL**
GERÄT ZUR BEHANDLUNG VON DIABETES IN EINEM SÄUGETIER
APPAREIL POUR LE TRAITEMENT DU DIABETE CHEZ UN MAMMIFERE

(30) Priority: 10.09.2004 ZA 200407280
(43) Date of publication of application: 11.07.2007
(73) Proprietor: Power of Grace (PTY) Limited, Lynwood Galeries, 0081Lynnwood (ZA)
(72) Inventor: JACOBUS DE VILLIERS, Hendrick, Waverly Pretoria 0186 (ZA)
(74) Representative: Frost, Alex John
(86) International application number: PCT/IB2005/002680
(87) International publication number: WO 2006/027679

(56) References cited:
- US-A- 4 844 075
- US-A- 5 458 626
- US-A- 5 851 223

## Description

### FIELD OF THE INVENTION

This invention relates to an apparatus for the treatment of diabetes in a mammalian patient.

### BACKGROUND TO THE INVENTION

Diabetes is an illness with debilitating effects on sufferers. It is commonly accepted that diabetes occurs in two forms, type-1 diabetes or early onset diabetes and type-2 diabetes or adult onset diabetes.

Diabetes is characterised by an inability of the body to convert sugar in a patient's blood to usable energy or to store it for later use. This leads to a high concentration of sugar in the patient's blood. The effects of this include reduced energy levels and problems related to the high sugar concentration itself, such as circulatory problems and so forth. There are also several other side effects to diabetes.

The most commonly accepted explanation for the cause of type-1 diabetes is that a patient suffering from it is born with dysfunctional beta cells in his pancreas, which leads to insufficient production of insulin, which is needed to convert blood sugar into usable energy or store it. Patients suffering from type-1 diabetes are normally insulin dependant and need to be injected with specific amounts of insulin to convert sugar in their blood.

The most commonly accepted explanation for the cause of type-2 diabetes is that a patient suffering from it has a system that has become insensitive to insulin. Therefore, even though such a patient's pancreas produces insulin this does not convert all the sugar in his blood into usable energy or store it which leads to the same problems as discussed above. Obesity is widely recognised as cause of type-2 diabetes. Patients suffering from type-2 diabetes are normally treated with insulin pills to supplement their existing insulin production and these patients are also encouraged to loose weight.

Diabetes sufferers, especially type-1 diabetes sufferers, normally face a lifetime of treatment including insulin injections which have to take place several times a day. This may include short-term insulin injections around meals and a long-term insulin injection that operates to provide a steady base for a whole day. Even so the onset of diabetes is normally a life altering event for a person.
Document US-A-5 851 223 discloses an apparatus according to the preamble of claim 1.

### OBJECT OF THE INVENTION

It is an object of this invention to provide an apparatus that at least partly overcomes the mentioned problems.

### SUMMARY OF THE INVENTION

In accordance with this invention there is provided an apparatus for use in the treatment of diabetes in a mammalian patient including means to generate a combined electric output signal and means to connect the combined output signal to a patient; the combined output signal being generated by summing at least two frequency modulated square wave signals having different unmodulated frequencies and which have been modulated by the same amount of frequency modulation.

There is also provided for the apparatus to include means to generate the unmodulated square wave signals, for the unmodulated frequency of the first square wave signal to be greater than the unmodulated frequency of the second square wave signal by a ratio of between about 6.7:1 and about 1.2:1, preferably by a ratio of between about 2.7:1 and about 2.3:1, further preferably by a ratio of about 2.5:1.

There is further provided for the apparatus to include means to generate an unmodulated first square wave signal having a frequency between 100 kHz and 200 kHz, preferably between about 149.846 kHz and 157.846 kHz, further preferably about 153.846 kHz; and means to generate an unmodulated second square wave signal having a frequency between 30 kHz and 85 kHz, preferably between about 58.5 kHz and 66.5 kHz, further preferably about 62.5 kHz.

There is still further provided for the apparatus to include means to modulate the first and second square wave signals, preferably by means of a modulating signal generator which generates a modulating square wave signal with a frequency of between 300 Hz and 350 Hz, preferably about 321 Hz.

According to a further feature of the invention there is provided for the apparatus to include means to generate a single output signal comprising an unmodulated square wave signal having a frequency of between 12Hz and 50Hz, preferably a frequency of about 12Hz; and for the apparatus to include means to connect the single output signal to a patient, preferably through the connecting means of the combined output signal.

There is also provided for the square wave signals to have amplitudes which range between 5V and 15V.

There is further provided for the connecting means to comprise at least one contact pad preferably having a diameter of at least 60mm, further preferably about 80mm.

There is still further provided for the apparatus to include timing means configured to alternately generate the combined output signal and single output signal for limited periods of time, preferably less than about 40 minutes, further preferably for about 20 minutes, still further preferably for about 10 minutes for each of the combined output signal and single output signals respectively.

There is also provided for the timing means to be configured to generate the single and combined output signals in succession and for the single output signal to be generated before the combined output signal.

### BRIEF DESCRIPTION OF THE DRAWINGS

An embodiment of the invention will be described below by way of example only and with reference to the accompanying representations in which:

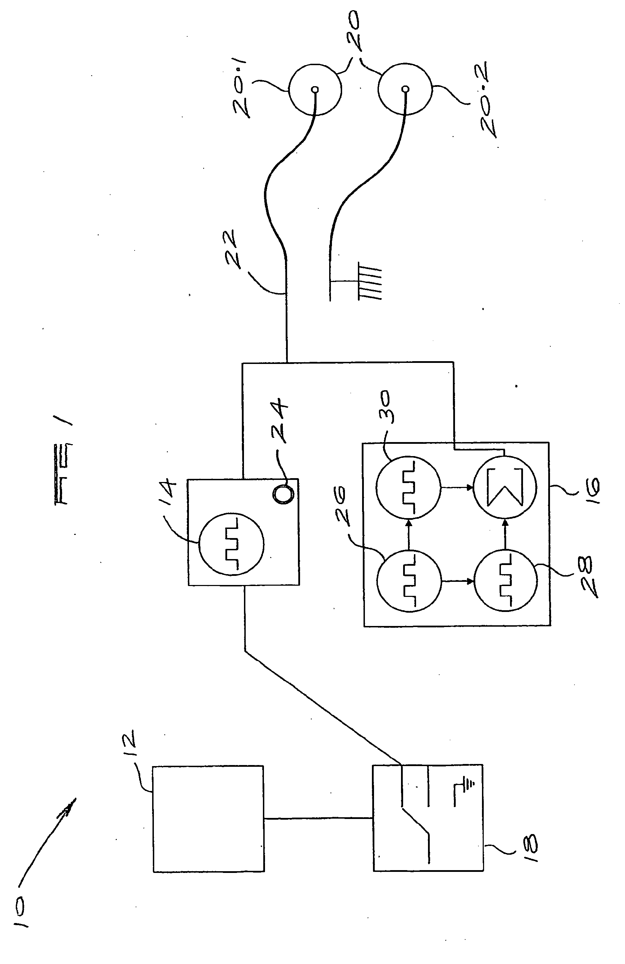

- Figure 1: is a diagrammatic representation of an apparatus according to the invention.

### DETAILED DESCRIPTION OF THE INVENTION

Referring to figure 1, the apparatus (10) has a timer (12), a 12 Hz signal generator (14), a combined output signal generator (16), a selector (18) and connectors (20).

The 12 Hz signal generator (14) generates a square wave (not shown), with a duty cycle of less than about 50% at a frequency of about 12 Hz. The 12 Hz square wave signal is applied to an output terminal (22) which in turn is connected to the body of a patient (not shown) through the connectors (20). The connectors (20) are preferably connected to the abdominal cavity or thorax of the patient. The connectors (20) consist of two standard conductive pads (20.1, 20.2) each pad having a diameter of about 80mm. A conductive gel is applied to the contacting side of each pad and the pads are then placed spaced apart on the abdomen of the patient. The 12 Hz signal generator (14) is also fitted with a potentiometer (24) through which a patient may adjust the amplitude of the 12 Hz square wave signal so as to achieve a comfortable treatment level.

The combined output signal generator (16) has a first square wave generator (26) which creates a modulating square wave at a duty cycle of about 60% and a frequency of about 321 Hz. A first square wave generator (28) is configured to produce a first square wave signal at a frequency of about 153.846 kHz and a duty cycle of about 60%. A second square wave generator (30) is configured to produce a second square wave signal at a frequency of about 62.5 kHz and a duty cycle of about 60%.

The square wave signals are generated to have amplitudes between 5V and 15V.

The 321 Hz square wave signal is fed into both the second and third square wave generators (28, 30) acting as a modulating signal which frequency modulates both the 153.846 kHz and 62.5 kHz signals. The output of the first square wave generator (28) is therefore a square wave having a frequency periodically varying between about 154.167kHz and 153.525kHz and the output of the second square wave generator (30) accordingly creates a square wave having a frequency periodically varying between about 62.821 kHz and 62.179kHz.

The outputs of the first and second square wave generators (28, 30) are then summed and applied to the output terminal (22) which, as described above, is then applied to the body of a patient through the connectors (20.1, 20.2).

The combination of the two frequency modulated signals created by the first and second square wave generators (28, 30) results in the creation of a wide range of harmonic frequencies.

The timer (12) is configured to provide a signal which toggles the selector (18) sequentially from a first to a second and then to a third state with a transition between states occurring only once the timer (12) has detected the lapse of a predetermined amount of time.

When the apparatus is switched on the selector (18) automatically moves to state one in which the 12Hz signal generator (14) is activated. After spending 10 minutes in state one the timer (10) actuates the selector (18) to move to state two. When the selector (18) is in state two, the combined output signal generator (16) is activated. After spending another 10 minutes in state two, the timer (12) again actuates the selector (18) to move to state three where the apparatus (10) switches off. The apparatus (10) is also provided with a reset button (not shown) which, when pressed, causes the selector (18) to move back to state one where the treatment cycle restarts.

It is believed that the application of the signal created by the combined output signal generator (16) to the body of a patient has definite medicinal advantages. In particular if the combined output signal is created using the specific base frequencies as described above and more particularly if the ratio between the base frequencies of the second and third square wave generators (28, 30) are as above, it is believed that application of the signal to the body of a patient is specifically advantageous as a treatment for diabetes.

It will be appreciated that this is only one embodiment of an apparatus according to the invention. Even though it is possible to produce the required output signals by means of electric circuitry different from that described here such different embodiments still fall within the scope of this invention.

It should also be noted that the duty cycle of 60% used in the example is not critical to the success of the treatment. Duty cycles varying between 10% and 60% have been tested and have not to affect the results significantly. It is also envisaged that duty cycles of higher than 60% may be used with success in this treatment.

## Claims

1. An apparatus for use in the treatment of a mammalian patient to at least partly stimulate regeneration of cells in the patient's body, the apparatus including means (16) to generate a combined electric output signal and means (20, 22) to connect the combined output signal to a patient; **characterized in that** the combined output signal being generated by summing at least two frequency modulated square wave signals having different unmodulated frequencies and which have been modulated by the same amount of frequency modulation.

2. An apparatus as claimed in claim 1 which includes means to generate the unmodulated square wave signals.

3. An apparatus as claimed in claim 1 or claim 2 in which the unmodulated frequency of the first square wave signal is greater than the unmodulated frequency of the second square wave signal by a ratio of between about 6.7:1 and about 1.2:1.

4. An apparatus as claimed in claim 1 or claim 2 in which the unmodulated frequency of the first square wave signal is greater than the unmodulated frequency of the second square wave signal by a ratio of between about 2.7:1 and about 2.3:1

5. An apparatus as claimed in claim 1 or claim 2 in which the unmodulated frequency of the first square wave signal is greater than the unmodulated frequency of the second square wave signal by a ratio of about 2.5:1.

6. An apparatus as claimed in any one of claims 1 to 5 which includes means to generate an unmodulated first square wave signal having a frequency between 100 kHz and 200 kHz, and means to generate an unmodulated second square wave signal having a frequency between 30 kHz and 85 kHz.

7. An apparatus as claimed in any one of claims 1 to 5 which includes means to generate an unmodulated first square wave signal having a frequency between 149.846 kHz and 157.846 kHz, and means to generate an unmodulated second square wave signal having a frequency between 58.5 kHz and 66.5kHz.

8. An apparatus as claimed in any one of claims 1 to 5 which includes means to generate an unmodulated first square wave signal having a frequency of about 153.846 kHz and means to generate an unmodulated second square wave signal having a frequency about 62.5 kHz.

9. An apparatus as claimed in any one of claims 1 to 8 which includes a modulating signal generator which generates a modulating square wave signal with a frequency of between 300 Hz and 350 Hz.

10. An apparatus as claimed in claim 9 in which the modulating signal generator generates a modulating square wave signal with a frequency of about 321 Hz.

11. An apparatus as claimed in any one of claims 1 to 10 which includes means to generate a single output signal comprising an unmodulated square wave signal having a frequency of between 12Hz and 50Hz.

12. An apparatus as claimed in claim 11 in which the single output signal has a frequency of about 12Hz.

13. An apparatus as claimed in claim 11 or claim 12 which includes means to connect the single output signal to a patient.

14. An apparatus as claimed in claim 13 in which the means to connect the single output signal to a patient comprises the connecting means of the combined output signal.

15. An apparatus as claimed in any one of claims 1 to 14 in which the square wave signals have amplitudes which range between 5V and 15V.

16. An apparatus as claimed in any one of the previous claims in which the connecting means comprises at least one contact pad.

17. An apparatus as claimed in claim 16 in which the contact pad has a diameter of at least 60mm.

18. An apparatus as claimed in claim 16, in which the contact pad has a diameter of about 80mm.

19. An apparatus as claimed in any one of the previous claims which includes timing means configured to alternately generate the combined output signal and single output signal for limited periods of time.

20. An apparatus as claimed in claim 19 in which the timing means is configured to alternately generate the combined output signal and single output signal for less than about 40 minutes for each of the combined output signal and single output signals respectively.

21. An apparatus as claimed in claim 19 in which the timing means is configured to alternately generate the combined output signal and single output signal for less than about 20 minutes for each of the combined output signal and single output signals respectively.

22. An apparatus as claimed in claim 19 in which the timing means is configured to alternately generate the combined output signal and single output signal for about 10 minutes for each of the combined output signal and single output signals respectively.

23. An apparatus as claimed in any one of claims 19 to 22 in which the timing means is configured to generate the single and combined output signals in succession and the single output signal is generated before the combined output signal.

## Patentansprüche

1. Vorrichtung zur Verwendung bei der Behandlung eines Säugetierpatienten, um wenigstens teilweise die Regeneration von Zellen im Körper des Patienten zu stimulieren, wobei die Vorrichtung ein Mittel (16) zum Erzeugen eines kombinierten elektrischen Ausgangssignals und ein Mittel (20,22) zum Verbinden des kombinierten Ausgangssignals zu einem Patienten umfasst,
**dadurch gekennzeichnet,**
**dass** das kombinierte Ausgangssignal durch Addition von wenigstens zwei frequenzmodulierten Rechtecksignalen erzeugt wird, die unterschiedliche unmodulierte Frequenzen aufweisen und die durch derselben Frequenzmodulationsbetrag moduliert worden sind.

2. Vorrichtung nach Anspruch 1, welche ein Mittel zum Erzeugen der unmodulierten Rechtecksignale umfasst.

3. Vorrichtung nach Anspruch 1 oder Anspruch 2, wobei die unmodulierte Frequenz des ersten Rechtecksignals um ein Verhältnis zwischen ungefähr 6,7:1 und ungefähr 1,2:1 größer als die unmodulierte Frequenz des zweiten Rechtecksignals ist.

4. Vorrichtung nach Anspruch 1 oder Anspruch 2, wobei die unmodulierte Frequenz des ersten Rechtecksignals um ein Verhältnis zwischen ungefähr 2,7:1 und ungefähr 2,3:1 größer als die unmodulierte Frequenz des zweiten Rechtecksignals ist.

5. Vorrichtung nach Anspruch 1 oder Anspruch 2, wobei die unmodulierte Frequenz des ersten Rechtecksignals um ein Verhältnis von ungefähr 2,5:1 größer als die unmodulierte Frequenz des zweiten Rechtecksignals ist.

6. Vorrichtung nach einem der Ansprüche 1 bis 5, welche ein Mittel zum Erzeugen eines unmodulierten ersten Rechtecksignals mit einer Frequenz zwischen 100 kHz und 200 kHz und ein Mittel zum Erzeugen eines unmodulierten zweiten Rechtecksignals mit einer Frequenz zwischen 30 kHz und 85 kHz umfasst.

7. Vorrichtung nach einem der Ansprüche 1 bis 5, welche ein Mittel zum Erzeugen eines unmodulierten ersten Rechtecksignals mit einer Frequenz zwischen 149,846 kHz und 157,846 kHz und ein Mittel zum Erzeugen eines unmodulierten zweiten Rechtecksignal mit einer Frequenz zwischen 58,5 kHz und 66,5 kHz umfasst.

8. Vorrichtung nach einem der Ansprüche 1 bis 5, welche ein Mittel zum Erzeugen eines unmodulierten ersten Rechtecksignals mit einer Frequenz von ungefähr 153,846 kHz und ein Mittel zum Erzeugen eines unmodulierten zweiten Rechtecksignals mit einer Frequenz von ungefähr 62,5 kHz umfasst.

9. Vorrichtung nach einem der Ansprüche 1 bis 8, welche einen Modulationssignalgenerator umfasst, welcher ein modulierendes Rechtecksignal mit einer Frequenz zwischen 300 Hz und 350 Hz erzeugt.

10. Vorrichtung nach Anspruch 9, wobei der Modulationssignalgenerator ein modulierendes Rechtecksignal mit einer Frequenz von ungefähr 321 Hz erzeugt.

11. Vorrichtung nach einem der Ansprüche 1 bis 10, welche ein Mittel zum Erzeugen eines einzelnen Ausgangssignals umfasst, weiches ein unmoduliertes Rechtecksignal mit einer Frequenz zwischen 12 Hz und 50 Hz umfasst.

12. Vorrichtung nach Anspruch 11, wobei das einzelne Ausgangssignal eine Frequenz von ungefähr 12 Hz hat.

13. Vorrichtung nach Anspruch 11 oder Anspruch 12, welche ein Mittel zum Verbinden des einzelnen Ausgangssignals mit einem Patienten umfasst.

14. Vorrichtung nach Anspruch 13, wobei das Mittel zum Verbinden des einzelnen Ausgangssignals mit einem Patienten das Verbindungsmittel des kombinierten Ausgangssignals umfasst.

15. Vorrichtung nach einem der Ansprüche 1 bis 14, wobei die Rechtecksignale Amplituden zwischen 5V und 15V aufweisen.

16. Vorrichtung nach einem der vorhergehenden Ansprüche, wobei das Verbindungsmittel wenigstens eine Kontaktunferlage umfasst.

17. Vorrichtung nach Anspruch 16, wobei die Kontaktunterlage einen Durchmesser von wenigstens 60 mm aufweist.

18. Vorrichtung nach Anspruch 16, wobei die Kontaktuntertage einen Durchmesser von ungefähr 80 mm aufweist.

19. Vorrichtung nach einem der vorhergehenden Ansprüche, welche ein Zeitmittel umfasst, das ausgelegt ist, das kombinierte Ausgangssignal und das einzelne Ausgangssignal abwechselnd für begrenzte Zeitspannen zu erzeugen.

20. Vorrichtung nach Anspruch 19, wobei das Zeitmittel ausgelegt ist, das kombinierte Ausgangssignal und das einzelne Ausgangssignal abwechselnd, für weniger als ungefähr 40 Minuten für jeweils das kombinierte Ausgangssignal und die einzelnen Ausgangssignale, zu erzeugen.

21. Vorrichtung nach Anspruch 19, wobei das Zeitmittel ausgelegt ist, das kombinierte Ausgangssignal und das einzelne Ausgangssignal abwechselnd, für weniger als ungefähr 20 Minuten für jeweils das kombinierte Ausgangssignal und die einzelnen Ausgangssignale, zu erzeugen.

22. Vorrichtung nach Anspruch 19, wobei das Zeitmittel ausgelegt ist, das kombinierte Ausgangssignal und das einzelne Ausgangssignal abwechselnd, für ungefähr 10 Minuten für jeweils das kombinierte Ausgangssignal und die einzelnen Ausgangssignale, zu erzeugen.

23. Vorrichtung nach einem der Ansprüche 19 bis 22, wobei das Zeitmittel ausgelegt ist, das einzelne und das kombinierte Ausgangssignal hintereinander zu erzeugen, und wobei das einzelne Ausgangssignal vor dem kombinierten Ausgangssignal erzeugt wird.

## Revendications

1. Appareil destiné à être utilisé dans le traitement d'un patient mammifère pour stimuler au moins partiellement la régénération de cellules dans l'organisme du patient, l'appareil comportant un moyen (16) destiné à générer un signal de sortie électrique combiné et un moyen (20, 22) destiné à connecter le signal de sortie combiné à un patient ; **caractérisé en ce que** le signal de sortie combiné est généré en sommant au moins deux signaux rectangulaires modulés en fréquence ayant des fréquences non modulées différentes et ayant été modulés avec le même niveau de modulation de fréquence.

2. Appareil selon la revendication 1, comportant un moyen destiné à générer les signaux rectangulaires non modulés.

3. Appareil selon la revendication 1 ou la revendication 2, dans lequel la fréquence non modulée du premier signal rectangulaire est supérieure à la fréquence non modulée du deuxième signal rectangulaire selon un rapport compris entre environ 6,7:1 et 1,2:1.

4. Appareil selon la revendication 1 ou la revendication 2, dans lequel la fréquence non modulée du premier signal rectangulaire est supérieure à la fréquence non modulée du deuxième signal rectangulaire selon un rapport compris entre environ 2,7:1 et environ 2,3:1.

5. Appareil selon la revendication 1 ou la revendication 2, dans lequel la fréquence non modulée du premier signal rectangulaire est supérieure à la fréquence non modulée du deuxième signal rectangulaire selon un rapport d'environ 2,5:1.

6. Appareil selon l'une quelconque des revendications 1 à 5, comprenant un moyen destiné à générer un premier signal rectangulaire non modulé ayant une fréquence comprise entre 100 kHz et 200 kHz, et un moyen destiné à générer un deuxième signal rectangulaire non modulé ayant une fréquence comprise entre 30 kHz et 85 kHz.

7. Appareil selon l'une quelconque des revendications 1 à 5, comprenant un moyen destiné à générer un premier signal rectangulaire non modulé ayant une fréquence comprise entre 149,846 kHz et 157,846 kHz, et un moyen destiné à générer un deuxième signal rectangulaire non modulé ayant une fréquence comprise entre 58,5 kHz et 66,5 kHz.

8. Appareil selon l'une quelconque des revendications 1 à 5, comprenant un moyen destiné à générer un premier signal rectangulaire non modulé ayant une fréquence d'environ 153,846 kHz et un moyen destiné à générer un deuxième signal rectangulaire non modulé ayant une fréquence d'environ 62,5 kHz.

9. Appareil selon l'une quelconque des revendications 1 à 8, comprenant un générateur de signal de modulation qui génère un signal rectangulaire de modulation ayant une fréquence comprise entre 300 Hz et 350 Hz.

10. Appareil selon la revendication 10, dans lequel le générateur de signal de modulation génère un signal rectangulaire de modulation ayant une fréquence d'environ 321 Hz.

11. Appareil selon l'une quelconque des revendications 1 à 10, comprenant un moyen destiné à générer un signal de sortie unique comprenant un signal rectangulaire non modulé ayant une fréquence comprise entre 12 Hz et 50 Hz.

12. Appareil selon la revendication 11, dans lequel le signal de sortie unique a une fréquence d'environ 12 Hz.

13. Appareil selon la revendication 11 ou la revendication 12, comprenant un moyen destiné à connecter le signal de sortie unique à un patient.

14. Appareil selon la revendication 13, dans lequel le moyen destiné à connecter le signal de sortie unique à un patient comprend un moyen de connexion du signal de sortie combiné.

15. Appareil selon l'une quelconque des revendications 1 à 14, dans lequel les signaux rectangulaires ont des amplitudes qui sont comprises entre 5 V et 15 V.

16. Appareil selon l'une quelconque des revendications précédentes, dans lequel le moyen de connexion comprend au moins une pastille de contact.

17. Appareil selon la revendication 16, dans lequel la pastille de contact a un diamètre d'au moins 60 mm.

18. Appareil selon la revendication 16, dans lequel la pastille de contact a un diamètre égal à environ 80 mm.

19. Appareil selon l'une quelconque des revendications précédentes, comprenant un moyen de cadencement configuré pour générer de façon alternée le signal de sortie combiné et le signal de sortie unique pendant des périodes de temps limitées.

20. Appareil selon la revendication 19, dans lequel le moyen de cadencement est configuré pour générer de façon alternée le signal de sortie combiné et le signal de sortie unique pendant moins d'environ 40 minutes pour chacun du signal de sortie combiné et des signaux de sortie uniques, respectivement.

21. Appareil selon la revendication 19, dans lequel le moyen de cadencement est configuré pour générer de façon alternée le signal de sortie combiné et le signal de sortie unique pendant moins d'environ 20 minutes pour chacun du signal de sortie combiné et des signaux de sortie uniques, respectivement.

22. Appareil selon la revendication 19, dans lequel le moyen de cadencement est configuré pour générer de façon alternée le signal de sortie combiné et le signal de sortie unique pendant environ 10 minutes pour chacun du signal de sortie combiné et des signaux de sortie uniques, respectivement.

23. Appareil selon l'une quelconque des revendications 19 à 22, dans lequel le moyen de cadencement est configuré pour générer les signaux de sortie unique et combiné consécutivement et dans lequel le signal de sortie unique est généré avant le signal de sortie combiné.
